# EUROPEAN PATENT APPLICATION

(11) **EP 3 878 972 A1**
(43) Date of publication of application: **15.09.2021**
(21) Application number: 20162489.7
(22) Date of filing: 11.03.2020
(51) Int. Cl.: C12Q 1/6806, C12Q 1/6869, C12Q 1/6876

(54) **BARCODING TECHNOLOGY FOR SEQUENCING OF NUCLEIC ACID MOLECULES**

(71) Applicant: UMC Utrecht Holding B.V., 3584 CM Utrecht (NL)
(72) Inventor: The designation of the inventor has not yet been filed
(74) Representative: V.O.

(57) **Abstract**

The invention relates to barcodes that have both unique and shared sequences. The barcodes are easy to sequence and to identify. In one aspect the invention provides a collection of barcoded nucleic acid molecules wherein each barcode comprises a sequence that is shared with the other barcodes in the collection and a sequence that is not shared with the other barcodes in the collection wherein the nucleotide residues that encode the non-shared sequence are interspersed with the nucleotide residues that encode the shared sequence. The invention also provides kits and method for sequencing nucleic acids.

## Description

The present invention generally relates to the field of nucleic acid sequencing. Some aspects relate to parallel sequencing wherein various nucleic acid molecules are sequenced in one reaction. The invention also relates to the barcoding of nucleic acid.

Next-generation sequencing (NGS), massively parallel sequencing, and high-throughput sequencing are related terms that describe innovation in DNA sequencing technology that allows for the sequencing of many nucleic acid molecules at the same time. Massively parallel sequencing processes create new requirements on the capability to discriminate sequence reads that are obtained in the sequencing process. Discrimination is, for instance, important for the correct alignment of reads from the same and similar templates, or to distinguish reads from different DNA samples, which are sequenced together on one sequencing chip, without physical separation between said samples.

NGS technology can be used in various ways. Whole-genome sequencing (WGS) and whole-exome sequencing (WES) being two examples. Targeted sequencing in cancer, for instance, remains advantageous for achieving very high coverage of the regions of interest while staying cost-effective and keeping the complexity of data interpretation manageable.

In parallel systems, it is often advantageous to attach tags or identifiers to the sequencing template so that later different reads of the same template are easily identified and grouped. This is particularly useful when a sample of nucleic acid contains repetitive or similar sequences, or when one has to discriminate between different molecules with (almost) the same sequence. Tagging individual templates with a barcode has been proposed and reported since at least 2007. In the past, barcodes or molecular indexes have been given various names such as unique identifiers (UID), unique molecular identifiers (UMI), primer ID, or duplex barcodes. Central to the definition is the unique sequence through which different barcodes, and nucleic acid molecules coupled to those barcodes, can be distinguished.

A barcode can be added to a template using several different techniques. One approach is to incorporate a barcode into sequencing adaptors during the library construction step for genome sequencing. This allows, for instance, "duplex sequencing", which greatly reduces errors by independently tagging and sequencing each of the two strands of a DNA duplex (Kennedy, S., Schmitt, M., Fox, E. et al. Detecting ultralow-frequency mutations by Duplex Sequencing. Nat Protoc 9, 2586-2606 (2014). Another approach is to incorporate a barcode into molecular inversion probes for targeted somatic mutation detection. Barcodes are also used in the molecular inversion probes and target-specific PCR primers. Barcodes are often produced synthetically by simultaneously incorporating different nucleotides while synthesizing oligonucleotides.

Barcoding is frequently used to tag original nucleic acid molecules in a sample. Nucleic acid collected form a tumor sample, for instance, typically contains nucleic acid from tumor cells and normal cells and the tumor cell nucleic acid may be heterogeneous with respect to the individual mutations in the DNA. This can lead to the sample having a number of nucleic acid molecules encoding a similar but different sequence. Associating such molecules with a unique identifier can help distinguishing differences present at the start of the sequencing from differences that occur as a result of sequence and/or amplification errors.

Barcodes are usually designed as a string of nucleotides, in some instances as a string of random nucleotides (such as the 7 nucleotide stretch NNNNNNN), or partially degenerate nucleotides (such as the 7 nucleotide stretch NNN(A/G)N(C/T)N), where N can be any nucleotide, or defined nucleotides (useful when template molecules are limited). Barcodes allow, among others, for the accounting of sequencer and PCR errors in high coverage NGS data. Barcodes can be assigned different names such as molecular barcodes, sample barcodes etc, depending on the exact application. For instance, where molecular barcodes are typically used to identify different molecules in a sample, sample barcodes are used to distinguish between different samples. The latter is, for instance, useful when samples are mixed prior to the sequencing of the DNA therein.

The present invention describes special barcodes and general methods to design and implement barcodes in, for instance, nucleic acid sequencing. The invention is particularly useful for sequencing technologies in which the signal given by a nucleotide is influenced by the one or more surrounding nucleotides. For example, Nanopore sequencing, particularly Oxford Nanopore sequencing technology, reads DNA as k-mers, where each stretch of a few consecutive bases (k-mer) of nucleic acids determine the change in electronic current that is measured when a nucleic acid molecule translocates through a nanopore (Rang et al, Genome Biology volume 19, Article number: 90 (2018)). The k-mer that determines each signal state can be 2 nucleotides, 3 nucleotides or 4 or more nucleotides, depending, among others, on the nature of the nanopore used for sequencing. Oxford Nanopore flow cells as presently commonly used for Nanopore sequencing utilizes k-mer of 5 to 8 nucleotides. Oxford Nanopore sequencing involves passage of a single nucleic acid strand through a nanopore. Nucleic acid molecules are not read base-by-base like, for instance, nanopore sequencing technologies developed by Genia/Roche, or Northshore Bio. Each k-mer has a distinct electronic signal state. From the signal states of different consecutive and overlapping k-mers, the base sequence can be determined by basecalling software, such as hidden markov models or recurrent neural networks. A single signal state, representing a single k-mer, can be assigned to a single base, but this is an artificial assignment to facilitate visualization of the connection between the nanopore electronic signal (k-mer signal states) and the accompanying base measurements. This artificial connection between electronic signal and individual nucleic acid bases is also used in the examples described herein. As a consequence of the measurement of k-mers, the detection of a single base is influenced by neighboring bases. For example, an A nucleotide may be represented by a different electronic signal when present in the k-mer GCAGA, then when, for example, the A nucleotide is present in the k-mer TAATG. This effect of neighboring bases on the measurement of a single base affects the discrimination of barcode nucleic acid sequences because the context of a base determines how well it can be measured and used as identifier for a barcode sequence. For sequencing methods reading nucleic acids based on k-mers, new methods for barcode design are needed for accurate reading of barcode nucleic acid sequences and subsequent assignment of a barcode to an individual nucleic acid molecule, or a group of nucleic acid molecules belonging to a sample.

### SUMMARY OF THE INVENTION

In one aspect the invention provides a collection of barcoded nucleic acid molecules wherein each barcode comprises a sequence that is shared with the other barcodes in the collection and a sequence that is not shared (i.e. also referred to as identifier sequence or bases) with the other barcodes in the collection,
wherein the nucleotide residues that encode the non-shared sequence are interspersed with the nucleotide residues that encode the shared sequence such that the barcode comprises at least one uninterrupted sequence with the general formula I:

Xr-Ym-Xs

wherein
X and Y are nucleotide residues;
r, s and m are respectively the number of consecutive X and Y nucleotide residues;
m is 1, 2 or 3; and r and s are independently an integer of 1 or more; and
wherein the Xr and Xs nucleotide residues encode all or part of the shared sequence and the Ym nucleotide residues encode all or part of the non-shared sequence.
Note that '-' does not refer to a minus sign, but refers to a separator that is used for readability of the formula.
In one embodiment the barcode comprises two or more uninterrupted sequences with the general formula I. When two or more uninterrupted sequences make up the barcode, the sequence of Y nucleotides and/or the number of Y nucleotides in the two or more uninterrupted sequences is typically different. The uninterrupted sequences can be adjacent to each other or separated by one or more nucleotide residues. They can in fact be separated by many nucleotide residues.

In a preferred embodiment at least one uninterrupted sequence has the general formula II:

Xr - Ym - Xs - (Z - Yn - Xt)p

wherein X, Y, r, s and m have the meaning as indicated herein above and n is 1, 2 or 3; t is an integer of 1 or more, p is an integer of 1 or more, and Z is optional and when present is 1 or more non-barcode nucleotide residues. In one embodiment p is 2, 3, 4 or 5.

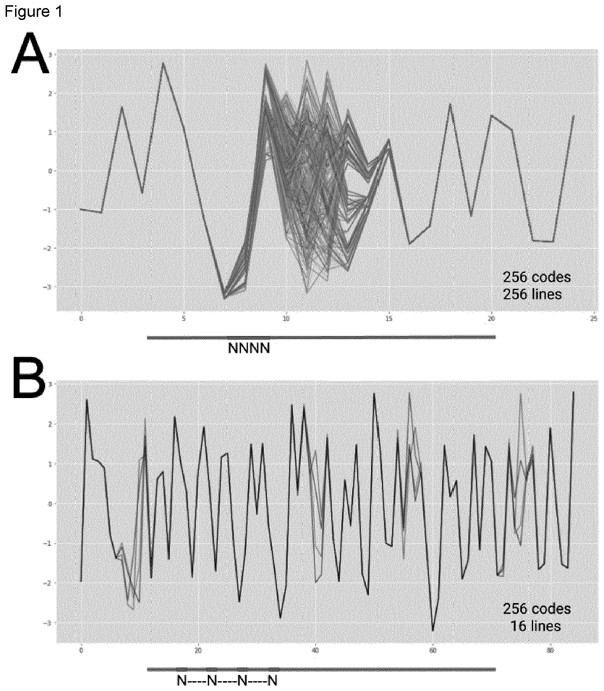

A barcode of the present invention can be read/identified more easily because the unique nucleotides are present in a stretch of known nucleotides of which the signal is known. When the number of adjacent Y nucleotides is more than 1 the signal of one may influence the signal of the other somewhat. An example of such an influence is depicted in Figure 1. When m=1, i.e. when there is one ,Y there are four different possible signals. Figure 1A shows the number of different signals when there are four consecutive Y nucleotides. In such a case there are 4x4x4x4 = 256 possible different signals. In one embodiment m=1 and the one or more of n, when present, are 1. In such cases the number of signal possibilities is reduced the most.

In one embodiment r and s are independently an integer of 5 or more and the one or more of t, when present, are independently 5 or more. The number of known/shared nucleotides that flank the variable Y nucleotide(s) is typically more when the signal measured for a Y nucleotide is also influenced by nucleotides that are not immediately adjacent to the Y nucleotide but are further away. Increasing the number of known/shared nucleotides adjacent to the Y nucleotide(s) decreases the influence of the Y signals on each other, thus facilitating the discrimination of the Y nucleotides. Having 5 or more X nucleotides is typically sufficient to limit the number of possible signals for Y from barcode to barcode.

A collection of barcoded nucleic acid molecules as described herein may further comprise one or more additional barcodes. The additional barcode can be a barcode as identified herein or another type of barcode.

The invention further provides a collection of at least two single or double stranded nucleic acid molecules that each comprise a head to tail repeat of a different member of the collection of barcoded nucleic acid molecules of the invention. Such head to tail concatemers are good substrates in long read sequencing methods. The barcodes in such substrates are well adapted for use in such long read sequencing methods. In such cases the head to tail repeat typically further comprises a stretch of nucleotides of which the sequence is to be determined.
The invention further provides examples of backbones with sparse barcodes. An example of a collection of nucleic acid molecules of the invention is a collection with the sequence GGGCATGCACAGATGTACACGAATCCCGAAGA**N**TGTTGTCCATTCATTGAATATGAGA TCTC**N**ATGGTATGATCAATAT**N**CGGATGCGATATTGATA**N**CTGATAAATCATATATGC ATAATCTCACATTATATTTATTATAATAAATCATCGTAGATATACACAATGTGAATTGTA TACAATGGATAGTATAACTATCCAATTTCTTTGAGCATTGGCCTTGGTGTAGATGCTGC ATGACATAGCCC, wherein the nucleotide N indicates the location of a Y nucleotide that can have any base, such a A, C, G, T. In a preferred embodiment the bases for the respective 4 Y nucleotides in this sequences are each A or T.
The invention thus further provides a method for sequencing a collection of nucleic acid molecules according to the invention. The sequencing method is preferably a long read sequencing method. The method preferably further comprises determining the sequence of the collection of nucleic acid molecules. The determination of the sequence preferably comprises aligning sequence reads on the basis of the sequence of the barcode in reads and determining a consensus sequence based on the alignment.

The invention further provides an algorithm to decode the barcodes from the raw Nanopore signal of a barcoded backbone.

The long read sequencing method is preferably Nanopore sequencing.

### BRIEF DESCRIPTION OF THE DRAWINGS

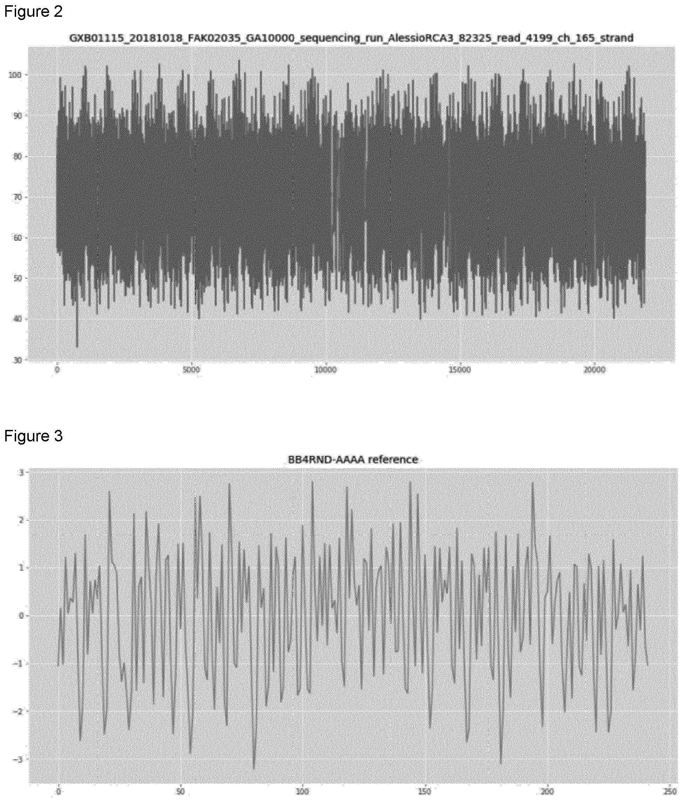

Figure 1. Simulated electric signal generated by Nanopore sequencing of 2 different type of DNA barcodes, both having four random nucleotides. In the barcode A, the random nucleotide are adjacent while in barcode B the random nucleotides are interspersed. Both barcodes can encode 256 tags (4^4). In the case of A (Sequence A), the signal of the random bases are influenced by each other causing a total of 256 different signals, many of which are overlapping and are difficult to discern. In the case of B (Sequence B) instead, the signal of a random base is not influenced by another, thus each position can be described by at most 4 discrete signals (16 in total, 4 per position), none of which is overlapping and thus much easier to discern.
   >Sequence A
      TCAATATCGGAT**NNNN**GCGATATTGATACT
   >Sequence B 
GAATCCCGAAGANTGTTGTCCATTCATTGAATATGAGATCTCNATGGTATGATCAATA
TNCGGATGCGATATTGATANCTGATAAATCAT
Figure 2. Electric signal recorded by the Nanopore MinION device for a concatemeric read. The unit of the Y axis is pico-ampere. On the X axis is represented the event number, which can be translated as base number in the read.
Figure 3. Normalized reference signal expected for the designed backbone (BB) sequence. In this case, the reference signal has all the Ns converted to A.
Figure 4. Alignment between the reference signal and the sample (measured) signal, both normalized. In this case, the reference signal has all N bases converted to A. The boxes highlight the position of the N bases.

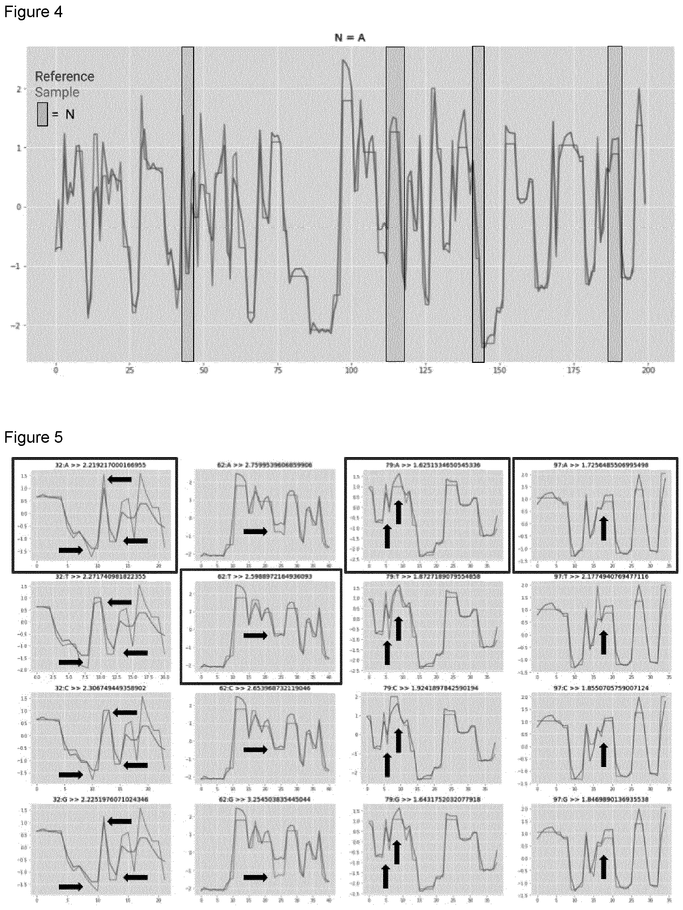

Figure 5. Visualization of intermediate steps of the barcode detection algorithm. Each column in the figure represents a specific position in the sequenced DNA (position number 32, 62, 79 and 97). For each position, a reference having one of the four DNA bases instead of the N is aligned to the measured signal and an alignment score is computed. For each position, the alignment with the lowest score is selected (highlighted with a bold box). The barcode sequence in this example is ATAA. Arrows point to visible differences between the alignments. Following this approach, the algorithm only need to perform 16 comparison (4*4) to detect any of the 256 (4^4) possible barcodes. Using a conventional barcode instead, one would require to compare the given signal with all the possible references (256 in this case) every time. Thus, the use of a sparse barcode reduces significantly the computation time needed to decode a barcode. Indeed, the computation time needed to decode a sparse barcode grows linearly with the number of random bases to be decoded, while in the case of a standard barcode, the computation time grows exponentially.
Figure 6. Consistency in barcode detection among concatemers with a GGAG barcode. Each column in the figure represents a segment in a concatemeric read.

For each of the N positions, an alignment score was calculated between the sequencing signal and the expected reference with one of the four bases assigned to N. The alignment score is then mapped to a color. Lighter color means lower alignment score, which means higher similarity between the sample signal and the reference.

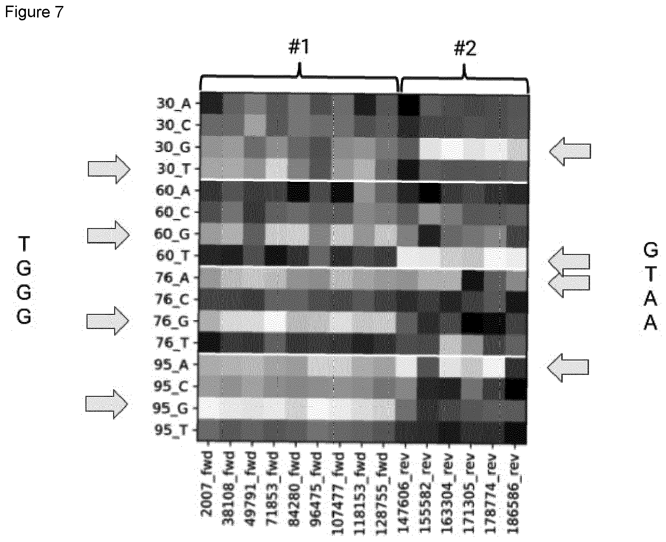

### Figure 7. Detection of hybrid concatemers.

Hybrid concatemers are formed when distinct concatemers are joined together via DNA ligation or when the signal generated by two distinct molecules is not correctly split in two reads by software that processes the raw sequencing signal, e.g. basecalling software or the like. Recognizing such artifacts is important for determining the sequencing of individual (single) nucleic acid molecules within a collection of nucleic acid molecules.

### DETAILED DESCRIPTION

A barcode of the present invention is particular suited for sequencing techniques in which the signal that a particular nucleotide gives when measured is influenced by the presence of one or more surrounding nucleotides. An example of such a technique is Oxford Nanopore sequencing as provided by Oxford Nanopore Technologies. Oxford Nanopore sequencing is a technique wherein the signal, typically an electrical current, is measured while a (single-stranded) nucleic acid molecule such as DNA or RNA is passed through a nanopore, typically a protein nanopore. At any given time multiple nucleotides are in the process of crossing the pore. The measured signal is typically a compilation of the signal produced by all of the nucleotides in or close to the pore. The sequence of the nucleic acid molecule that is passed through the pore is determined on the basis of the aggregate signals of the respective sets of nucleotides that is measured at any given time point. This is a time consuming step, even for a computer as the various signals are compared with historical signals and different sequences may give an almost identical signal. Assigning a nucleic acid sequence to a particular signal may thus be time consuming.

In such and similar cases, commonly used barcoding strategies, which make use of a stretch of consecutive bases with a random or predefined sequence, are difficult and computationally expensive to be decoded from the raw (nanopore) sequencing signal and generally prone to base calling errors, leading to barcode misassignment. Such barcoding strategies, which involve a stretch of consecutive bases, are not ideal for sequencing approaches where nearby bases influence the raw sequencing signal.

The invention disclosed herein provides a solution to these and other problems with sequence barcodes. In particular, a barcode design is provided that isolates the signal-confounding-factors. A series of algorithms able to decode the correct bases from the designed barcodes can be provided that only use a fraction of the computing power usually needed to decode common barcodes. Barcodes of the invention are less prone barcode misassignment errors. In contrast to common barcodes, formed by consecutive stretches of bases (---NNNN---) (where N is a non-shared base while the dash symbol "-" is a shared base) we, in one embodiment provide barcodes on which the unique/identifier barcode bases Ym are separated by shared bases X where X is preferably, at least, the number of nucleotides contributing to a given sequencing signal (e.g. 5 to 6 bases in the case of the current version Oxford Nanopore sequencing devices).

A barcode of the present invention comprises a sequence that is shared with other barcodes in the collection. This shared sequence is composed of nucleotide residues that are shared among the barcodes. Such nucleotides are represented by the letter X in the formulas I and II as described herein. Such nucleotides are also referred to as shared nucleotides, or non-identified nucleotides.
The barcodes also comprise a sequence that is not shared with other barcodes in the collection. The non-shared sequence is composed of nucleotides that together distinguish the barcode from other barcodes in the collection. Such nucleotides are represented by the letter Y in the formulas I and II as described herein. Such nucleotides are also referred to as identifier nucleotides or non-shared nucleotides. Such non-shared nucleotides together identify the barcode in the collection. Such nucleotide residues are also referred to as non-shared, or identifier bases or identified nucleotide residues and sometimes also as unique bases or unique nucleotide residues. Where strictly speaking it is the set of identified nucleotides in a barcode that together identify the barcode, where individual non-shared nucleotides may have identical residues in another barcode.

The terms "nucleotide(s)", "nucleotide residue(s)", "residues" and "bases" are often used interchangeably.

How many identifier bases are necessary to identify a nucleic acid molecule or a set of nucleic acid molecules such as for a sample barcode depends on the number of distinguishing events needed. In complex cases many different identifier nucleotides are desired, for instance when analyzing samples with hundreds of different nucleic acid molecules. In other instances already a few different identifier bases are sufficient. A barcode used may be more complex than required to identify different molecules, in such cases the non-shared nucleotides are still referred to as identifier bases or unique bases.

A collection of barcoded nucleic acid molecules comprises different nucleic acid molecules. The difference may only be in the sequence of the barcode in the respective molecules. This is useful, for instance, in preparing a cloning tool for capturing nucleic acid molecules from a complex mixture of molecules. Barcoded nucleic acid molecules that have captured one molecule from the mixture can be distinguished from barcoded nucleic acid molecules that have not captured or have captured a different nucleic molecule from the complex mixture. In various embodiments, the differences are more than only the sequence of the barcode. In the capture example above, the difference may, in addition to the sequence of the barcode, also be in sequence, size or both of the captured nucleic acid.

A collection of barcoded nucleic acid molecules can be a collection comprising different nucleic acid molecules where the different nucleic acid molecules comprise at least a barcode with a sequence that is different from the barcode sequence of other nucleic acid molecules in the collection.
A collection of barcoded nucleic acid molecules may consist of a few different nucleic acid molecules such as two, three, four or more, but typically it contains more than 10, preferably more than 20, 30, 40 preferably more than 50 different nucleic acid molecules.

A barcode may be RNA, DNA or a combination thereof. It may comprise all naturally occurring nucleotides but may also contain, or be composed entirely of, non-natural nucleotides. Canonical bases are the four C, T, G, A. Uracil (U) is another natural base. Such bases may be modified in a natural way such as 5-methyl cytosine found in CpG islands, or an inosine. Various non-natural bases are known in the art. Barcodes in a collection of nucleic acids may comprises one or more of such non-natural bases. Similarly nucleic acid molecules in a collection as described herein may comprises one or more of such non-natural bases.

Polynucleotide barcodes are well-known in the art (Kozarewa, I. et al, (2011), Methods Mol. Biol. 733, p279-298). In nanopore sequencing a barcode is a specific sequence of nucleotides that affects the current flowing through the pore in a specific manner. A nucleotide typically contains a nucleobase, a sugar and at least one phosphate group. The nucleobase is typically heterocyclic.

Nucleobases include, but are not limited to, purines and pyrimidines and more specifically adenine, guanine, thymine, uracil and cytosine. The sugar is typically a pentose sugar.

Nucleotide sugars include, but are not limited to, ribose and deoxyribose. The nucleotide is typically a ribonucleotide or deoxyribonucleotide. The nucleotide typically contains a monophosphate, diphosphate or triphosphate. Phosphates may be attached on the 5' or 3' side of a nucleotide.

Nucleotides include, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (HDP), uridine triphosphate (UTP), cytidine monophosphate (CMP), cytidine diphosphate (CDP), cytidine triphosphate (CTP), 5 -methyl cytidine monophosphate, 5- methylcytidine diphosphate, 5-methylcytidine triphosphate, 5-hydroxymethylcytidine monophosphate, 5-hydroxymethylcytidine diphosphate, 5-hydroxymethylcytidine triphosphate, cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP), 5-methyl-2' -deoxycytidine monophosphate, 5- methyl -2' -deoxycytidine diphosphate, 5-methyl-2' -deoxycytidine triphosphate, 5- hydroxymethyl-2'-deoxycytidine monophosphate, 5-hydroxymethyl-2'-deoxycytidine diphosphate and 5-hydroxymethyl -2' - deoxycytidine triphosphate. The nucleotides in the adaptor are preferably selected from AMP, TMP, GMP, UMP, dAMP, dTMP, dGMP or dCMP. The nucleotides may be abasic (i.e., lack a nucleobase). The nucleotides may contain additional modifications. In particular, suitable modified nucleotides include, but are not limited to, 2' amino pyrimidines (such as 2' -amino cytidine and 2' -amino uridine), 2'-hyrdroxyl purines (such as , 2'-fluoro pyrimidines (such as 2'-fluorocytidine and 2'fluoro uridine), hydroxyl pyrimidines (such as 5'-a-P-borano uridine), 2'-0-methyl nucleotides (such as 2'-0-methyl adenosine, 2'-0-methyl guanosine, 2'-0-methyl cytidine and 2'-0-methyl uridine), 4'-thio pyrimidines (such as 4'-thio uridine and 4'-thio cytidine) and nucleotides have modifications of the nucleobase (such as 5-pentynyl-2'-deoxy uridine, 5-(3-aminopropyl)-uridine and 1,6- diaminohexyl-N-5-carbamoylmethyl uridine).

One or more abasic nucleotides may be included in the barcode. Using one or more abasic nucleotides results in characteristic current spikes. This allows the clear highlighting of the positions of the one or more nucleotide species in the barcode.

The nucleotide species in the barcode may comprise a chemical atom or group such as a propynyl group, a thio group, an oxo group, a methyl group, a hydroxymethyl group, a formyl group, a carboxy group, a carbonyl group, a benzyl group, a propargyl group or a propargylamine group. The chemical group or atom may be or may comprise a fluorescent molecule, biotin, digoxigenin, D P (dinitrophenol), a photo-labile group, an alkyne, DBCO, azide, free amino group, a redox dye, a mercury atom or a selenium atom.

The barcode may comprise a nucleotide species comprising a halogen atom. The halogen atom may be attached to any position on the different nucleotide species, such as the nucleobase and/or the sugar. The halogen atom can be fluorine (F), chlorine (CI), bromine (Br) or iodine (I). In some embodiments the halogen atom is F or I.

Sequencing of nucleic acids may or may not involve the copying of a strand a nucleic acid. For Oxford Nanopore DNA sequencing, the nucleic acids are typically not copied as part of the sequencing step.. Nucleic acid molecules for sequencing, can be or be derived from an amplification reaction prior to determining the sequence thereof. barcode typically comprises a sequence encoded by nucleotides that can pair with the nucleotides used for the sequencing. Typically, though not necessarily such barcode nucleotides are C, T, G, A and/or U. In one embodiment of the invention, a unique barcode sequence may be represented by one or two specific bases instead of all the 4 possible bases. The four bases A, C, G, and T (U in case of RNA) not all influence the detection signal in the same way. Bases used for encoding the different barcode sequences in a barcode are in some embodiments chosen among the ones affecting the signal in a way that is easily distinguishable. For example, in the case of Oxford Nanopore sequencing, A and T affect the signal in opposite ways, T increases the measured current while A decreases it. As a consequence, a barcode wherein the complexity is encoded by specific sequence/combinations with As and Ts are typically easier to discriminate. Such barcodes may have the formula (---Y-----Y-----Y-----Y---) wherein the respective Y are independently A or T and "-" is a barcode base that is shared (X) among the barcodes.

The number of interspersed non-shared barcode bases (Y) can be 1 or more. Barcoding is typically done to distinguish more than 4 different nucleic acid molecules. The number of Y nucleotides can be 2, 3, 4, 5 or more. For complex mixtures the number of barcode nucleotides is sufficient to encode an adequate number of the nucleic acid molecules. In one embodiment of the invention Y can represent one or two specific bases instead of all the 4 possible bases, usually chosen among the ones affecting the signal in a way that is easily distinguishable. For example, in the case of the present Oxford Nanopore sequencing technology we noticed that A and T affect the signal in opposite ways, T increases the measured current while A decreases it. As a consequence, if the barcode is composed of a specific sequence/combination with As and Ts (---Y-----Y-----Y-----Y---) (Y = A or T), it is easier to discriminate the correct base.

A barcode preferably has at least three nucleotide residues the sequence of which identifies a nucleic acid molecule. The nucleic acid molecule is said to have 3 non-shared, identifier or unique nucleotides, nucleotide residues or bases. The terms "non-shared", "identifier" and "unique" are here used interchangeably.

What nucleotides form part of the identifier nucleotides is typically apparent from the position of the nucleotide in a nucleic acid or nucleic acid sequence. The sequence of YYYY in the following sequence:

| | |
|---|---|
| Barcode nucleotides | ---Y-----Y-----Y-----Y--- |
| Position | ---4-----10----16----22-- |

can be identified easily through the position of the nucleotides in the sequence. In the above example the barcode comprises the sequence of the nucleotides in positions 4, 10, 16 and 22.

The identifier nucleotide residues are interspersed with nucleotide residues that are shared with other barcodes. Such nucleotide residues are also called non-identifier residues. A barcode preferably comprises at least one stretch of at least 5 consecutive nucleotide residues comprising one or two identifier residues and at least three or four non-identifier residues. Preferably the collection of nucleic acid molecules comprises at least two different stretches of at least 5 consecutive nucleotide residues comprising one or two identifier residues and at least three or four non-identifier residues. A barcode preferably comprises at least four different stretches of 5 consecutive nucleotide residues comprising one or two identifier residues and at least three or four non-identifier residues. The stretches of at least 5 consecutive residues are preferably non-overlapping stretches.

In one embodiment at least two identifier residues are separated by at least 5 non-identifier residues. In a preferred embodiment at least three identifier residues each are separated from another identifier residue by at least 5 non-identifier residues. In embodiments expressing a residue separation or minimum residue distance between two identifier residues, there are no other identifier residues in between.

In embodiments comprising 2 or more identifier residues it is preferred that at least two identifier residues are flanked on either side by at least 5 non-identifier residues. In embodiments wherein the barcode comprises more than 2 identifier residues it is preferred that all of the discriminative identifier residues are flanked on either side by at least 5 non-identifier residues. It is preferred that an identifier residue is flanked on either side by at least 5 non-identifier residues. For different (Nanopore) sequencing chemistries, the number non-identifier bases may be adapted according to the length of each k-mer that is read by the sequencer. In such cases, the number of non-identifier residues may also be 4 or less.

In one embodiment the barcode comprises 4 identifier residues and at least 25 non-identifier residues and each identifier residue is flanked on either side by a stretch of at least 5 non-identifier residues. The non-identifier residues preferably have a sequence that is shared with the nucleic acid molecules in the collection.

In one embodiment the nucleic acid molecules in the collection of barcoded nucleic acid molecules are circular molecules. In one embodiment the circular nucleic acid molecules are double stranded circular nucleic acid molecules.

In a further embodiment the nucleic acid molecules of the collection are double stranded nucleic acid molecules.

A barcode of the invention can be used for sequencing as such. A barcode of the invention is particularly suited for sequencing concatemers. Such concatemers can be produced among others by rolling circle amplification of circular nucleic acid molecules. The presence of a repeated barcode can help in the alignment of sequences, in attributing sequence reads to a certain physical molecule and thereby aid in determining the sequence of the original circular nucleic acid molecule and resolving hybrid reads as depicted in Figure 7. It also aids in discriminating between sequencing error, be it read errors or mutations or otherwise. Thus in an embodiment at least one of the nucleic acid molecules comprises two or more identical barcodes. In an embodiment at least one of the nucleic acid molecules comprises a plurality of concatemers. In an embodiment, the nucleic acid molecules in the collection are linear. In an embodiment, the nucleic acid molecules in the collection do not have a concatemer. In one embodiment the barcode of the present invention is at one end of the linear nucleic acid molecules in the collection. In another embodiment the barcode is at both ends of the linear nucleic acid molecules in the collection. A collection of nucleic acid molecules as described herein can have circular and linear molecules; single and double stranded molecule; molecules comprising concatemers and molecules that do not have concatemers; or combinations thereof. In one embodiment the collection of nucleic acid molecules comprises linear nucleic acid that do not have concatemers and that have the barcode at one or both ends of the molecules.

Nucleotide residue(s) that encode the non-shared sequence are interspersed with the nucleotide residues that encode the shared sequence. This is such that the barcode comprises at least one uninterrupted sequence with the general formula I:

Xr-Ym-Xs

wherein
X and Y are nucleotide residues;
r, s and m are respectively the number of consecutive X and Y nucleotide residues;
m is 1, 2 or 3; and r and s are independently an integer of 3 or more; and
wherein the Xr and Xs nucleotide residues encode all or part of the shared sequence and the Yn nucleotide residues encode all or part of the non-shared sequence. In a preferred embodiment at least one uninterrupted sequence has the general formula II:

   Xr - Ym - Xs - (Z - Yn - Xt)p

   wherein X, Y, r, s and m have the meaning as indicated herein above and n is 1, 2 or 3; t is an integer of 1 or more and p is an integer of 1 or more. In one embodiment p is 2, 3, 4 or 5.

Z is optional and when present can be one or more nucleotide residues that are not part of the barcode. When Z is present and p is 2 or more, Z can be present in the first instance of p or the second instance of p or both. Similarly for values of p of higher than 2. Z may be present once, twice, three times. The number of Zs can of course not exceed the value of p but can easily be smaller that p but at least 0.

The number of X nucleotide residues in the general formulas I and II are represented by the variables r, m, s and t. The values for r, m, s and t are preferably at least 1, more preferably at least 2, more preferably at least 3. In a particularly preferred embodiment the values are 4 or 5, or at least 4 or at least 5. With increasing number of shared nucleotides between identifier nucleotide(s) the complexity of the identifier signal become less. This helps with the identification of the identifier nucleotide(s) and the time needed to identify the identifier nucleotide(s). The respective values for r, m, s, and t may be all the same or different. For instance, in one case r = m = s = t = 1, or r = m = s = t = 2, r = m = s = t = 3, r = m = s = t = 4, r = m = s = t = 5. In other cases r is not m is not s is not t.

The number of consecutive Y nucleotides is preferably 1, 2 or 3. In other words the value of the variable m or n is 1, 2, or 3. These values are independent from each other. Where m may be 2, n may be 1 or where m may be 1, a first of n can be 1 and a second of n can be 2. The latter embodiment may occur when p has a value of 2 or more. The value of p is preferably at least 2, more preferably at least 3, more preferably at least 4, more preferably at least 5, more preferably at least 6.

Suitable embodiments for the values of the respective values for the variables in the formulas I and II are also indicated in table 2.

The barcode sequence that is not shared with other barcodes in the collection can be used to identify a certain nucleic acid molecule that comprises this barcode. The actual nucleotide residues that make up this non-shared sequence is interspersed with nucleotide residues that are shared with other barcodes in the collection. The value of p in general formula II: Xr - Ym - Xs - (Z - Yn - Xt)p is at least 1. The general formula I Xr - Ym - Xs can be seen as the instance where the value of p = 0. In case of p being not 0, the non-shared nucleotide sequence represented by the variables Ym and Yn is interrupted by at least Xs. When reference is made to an uninterrupted sequence reference is made to a continuous uninterrupted sequence of adjacent nucleotides.

In one embodiment the invention provides a collection of nucleic acid molecules comprising two or more collections of barcoded nucleic acid molecules of the invention. Molecules of the invention preferably further comprise a further sequence of about 20-1000 nucleotides in addition to the sequence of the barcode. The further sequence can be the target nucleic acid of which the sequence is to be determined and/or be another sequence present for another purpose. Non-limiting other functions of sequence are indicated herein below.
The nucleic acid molecules of the collection can have a length to code for at least a barcode of formula I. Typically the length is at least 20 nucleotides. The nucleic acid molecules of the collection are suitably on average between 150 and 4000 nucleotides long. Such average length includes the length of the barcode and the length of the additional sequence. In one embodiment the molecules are circular molecules, preferably double stranded circular molecules. Circular molecules are suitable substrates for rolling circle amplification (RCA). RCA describes a process of nucleic acid replication that can rapidly synthesize multiple copies of circular molecules of DNA or RNA, such as plasmids, the genomes of bacteriophages, and the circular RNA genome of viroids. The process typically is started by providing single strand circles or nicked double stranded circles and providing a replication initiation site such as an oligonucleotide primer or the mentioned nicked template. The amplification can be initiated by adding a polymerase under suitable reaction conditions. The polymerase will start the replication and can continue on when it has completed one cycle. Special polymerases are available that allow extension of the amplicon to cover many copies of the circle.
As a simplified version of natural rolling circle replication, an isothermal DNA amplification technique, rolling circle amplification (RCA) was developed. The RCA mechanism is widely used in molecular biology & biomedical nanotechnology. Since its initial discovery, many variations of RCA have been developed.

The product or amplicon of an RCA reaction is typically a long typically single stranded nucleic acid molecule comprising various copies of the circle. Such an amplicon thus typically has two or more identical barcodes. In one embodiment the invention thus provides a collection of barcoded nucleic acid molecules as described in the present invention comprising two or more nucleic acid molecules that have two or more identical barcodes as described herein.

The barcoded nucleic acid molecules in a collection of the invention may be double stranded, single stranded or a combination thereof.

In one embodiment the nucleic acid molecules in a collection of the invention are circular molecules.

Further provided is a method for determining the sequence of a collection of nucleic acid molecules comprising providing a collection of barcoded nucleic acid molecules of the invention, amplifying said collection of nucleic acid molecules, sequencing the amplified nucleic acid in parallel and aligning sequencing results on the basis of the sequence of the barcode and determining the sequence of the collection of nucleic acid molecules based on the alignment. In a preferred embodiment the sequencing comprises long-read sequencing. The sequencing is preferably nanopore sequencing.

In the case of Oxford nanopore sequencing, for example with R9.4.1 pore chemistry, the signal is given by the interaction of a few nucleotides, typically 5-6 nucleotides inside the pore, so the bases of our barcodes will be spaced at least 5-6 nucleotides apart (---Y-----Y-Y-----Y---) which we call a "an interspersed identifier residue". With such a design, the signal of each Y base is not affected by any other Y base, but only by non-random bases depicted by the dash symbol "-", which enables computationally efficient and accurate detection of the barcode attached to every sequenced molecule. For other versions of Nanopore sequencing, the identifier nucleotides may be spaced by more than 5-6 nucleotides, or less than 5-6 nucleotides, depending on the nature of the sequencing signal.

The reference to a barcode in the singular is typically used to indicate a region in a collection of nucleic acid molecules that is reserved or designated for introducing barcode nucleotides. Such a barcode is typically used for a specific purpose, for instance as a molecular barcode. A molecular barcode is typically used to identify a particular molecule and copies and sequences thereof. A sample barcode is typically used to identify nucleic acid molecules derived from one sample and copies and sequences thereof. The plural "barcodes" is typically used to refer to different barcodes or occasionally to different sequences/occurrences of a barcode in one collection. The context of the sentence makes the different use clear. Different barcodes in the sense that different coding information is incorporated can be in the same region or in a different region reserved for or designated for introducing barcode nucleotides. In the present invention the non-identifier nucleotides are interspersed with identifier nucleotides. The non-identifier nucleotides typically have a sequence that is shared with other nucleic acid molecules that have a different identifier sequence. The non-identifier nucleotides have a function in separating the identifier nucleotides from each other. They can also have other functions. One such other function can be a backbone function to capture target nucleic acid. Further functions for such other nucleotides are the presence of a convenient restriction enzyme site, a suitable site for targeting a sequence or amplification primer, a promoter binding site and a transcription factors binding site. In general, any protein binding site. It can also have the function of a G-quadruplex forming sequence, or be a sequence useful for pull down or affinity purification. The skilled person can readily identify other functions of sequences suitable for incorporation in a collection of nucleic acid molecules as described herein. The list specified here is thus not limiting.

For the purpose of clarity and a concise description features are described herein as part of the same or separate embodiments, however, it will be appreciated that the scope of the invention may include embodiments having combinations of all or some of the features described.

### EXAMPLES

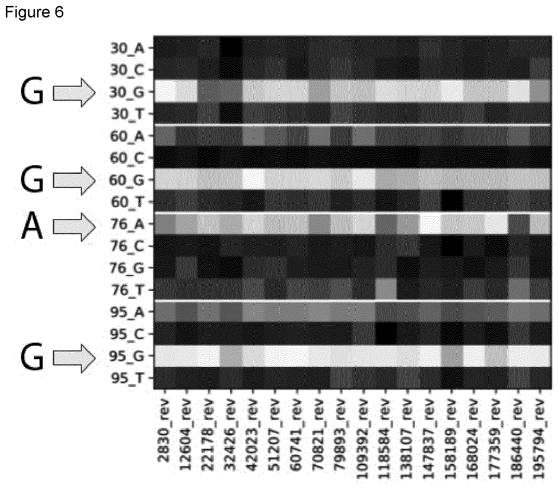

The barcoding technology described in this application can be used for instance to detect hybrid concatemers. The analysis of the barcode detected on the particular read of Figure 7 shows a first molecule having barcode TGGG connected to a molecule with barcode GTAA. The barcode can information can be used to correctly split the read and increase the sequencing accuracy of this run. In Figure 6 it is shown that as expected, the barcode of each repeat of the concatemer sequence is consistently decoded.

### Generation of concatemers

Concatemers are synthesized via rolling circle amplification (RCA) of circular DNA template.
The circular template was obtained by intramolecular self-ligation of a double stranded and blunt-ended DNA.

The initial linear DNA was generated by annealing followed by fill-in of the following "top" and "bottom" phosphorylated oligos:
>top 
GGGCATGCACAGATGTACACGAATCCCGAAGANTGTTGTCCATTCATTGAATATGAGA
TCTCNATGGTATGATCAATATNCGGATGCGATATTGATANCTGATAAATCATATATGC
ATAATCTCACATTATATTTA
>bottom

GGGCTATGTCATGCAGCATCTACACCAAGGCCAATGCTCAAAGAAATTGGATAGTTATA
CTATCCATTGTATACAATTCACATTGTGTATATCTACGATGATTTATTATAATAAATATA
ATGTGAGATTATGCATA

The fill-in reaction was mediated by a proof-reading DNA Polymerase, specifically Phusion DNA Polymerase, leading to the blunt-ended product "BB" (247bp long).
>BB

GGGCATGCACAGATGTACACGAATCCCGAAGANTGTTGTCCATTCATTGAATATGAGA
TCTCNATGGTATGATCAATATNCGGATGCGATATTGATANCTGATAAATCATATATGC
ATAATCTCACATTATATTTATTATAATAAATCATCGTAGATATACACAATGTGAATTGTA
TACAATGGATAGTATAACTATCCAATTTCTTTGAGCATTGGCCTTGGTGTAGATGCTGC
ATGACATAGCCC

The product (BB) was gel purified and used as input in a classic ligation reaction using T4 DNA Ligase.
Low concentration of BB in the reaction (5ng/ul) was used to increase the chance of self-circularization.

Linear-DNA was then removed using Lambda Exonuclease.

The circular DNA left was used as input for the RCA reaction, mediated by Phi29 DNA Polymerase.

### Sequencing

The RCA product was sequenced using a Nanopore MinION device following the protocol provided by the vendor.

### Data analysis

The expected raw sequence is generated for 4 versions of the BB; filling the N positions with only A, C, T, or G, in each of them. For example:
>BB:A

GGGCATGCACAGATGTACACGAATCCCGAAGAATGTTGTCCATTCATTGAATATGAGA
TCTCAATGGTATGATCAATATACGGATGCGATATTGATAACTGATAAATCATATATGC
ATAATCTCACATTATATTTATTATAATAAATCATCGTAGATATACACAATGTGAATTGTA
TACAATGGATAGTATAACTATCCAATTTCTTTGAGCATTGGCCTTGGTGTAGATGCTGC
ATGACATAGCCC

The expected raw sequences are mapped to the actual raw read data. At (and around) the locations of the random base pairs, a fitness score is calculated, describing how well the expected raw sequence fits the reads actual raw data at that position. Doing this for all possible bases at all randomized positions, a distance to each possible base is calculated for all randomized positions. This result is shown in the Figure 5, where each column shows the results for every N nucleotide present in 4 different positions (32, 62, 79, 97), and in each row, the N nucleotide is compared to all possible reference signals, A, T, C, G.
After normalization of these distances, direct comparisons between backbones are possible (i.e. distance between groups of backbones) and changes in the backbone's barcode can be detected measuring the distances to all possible random bases change. The match with the lowest distance is then chosen as the barcode sequence. In figure 5, the resulting barcode is ATAA.

**Table 1 lists the IUPAC letter coding for nucleotides. Unless otherwise specifically mentioned the letter coding as used herein does not follow the IUPAC coding. For example the use of the letter code "Y" in the claims does not refer to the nucleotides only C or T but to any nucleotide.**

| IUPAC nucleotide code | Base |
|---|---|
| A | Adenine |
| C | Cytosine |
| G | Guanine |
| T (or U) | Thymine (or Uracil) |
| R | A or G |
| Y | C or T |
| S | G or C |
| W | A or T |
| K | G or T |
| M | A or C |
| B | C or G or T |
| D | A or G or T |
| H | A or C or T |
| V | A or C or G |
| N | any base |
| . or - | gap |

**Table 2. The table lists embodiments with various combinations of values for the respective variables of formula I and formula II. Z can be present or absent. The values for n and t are given for each of p. For example, when p=2 then both occurrences of n and both occurrences of t have the indicated p value in the table. Z in this case can be present or absent in both occurrences or a combination thereof, present in the first occurrence and absent in the second or the other way around. The same values for n, t, and presence or absence of Z or any combination thereof are indicated for higher values of p.**

| r | m | s | n | t | p |
|---|---|---|---|---|---|
| 1 | 1 | 1 | 1 | 1 | 1 |
| 1 | 1 | 1 | 1 | 1 | 2 |
| 1 | 1 | 1 | 1 | 1 | 3 |
| 1 | 1 | 1 | 1 | 1 | 4 |
| 1 | 1 | 1 | 1 | 1 | 5 |
| 2 | 1 | 2 | 1 | 2 | 1 |
| 2 | 1 | 2 | 1 | 2 | 2 |
| 2 | 1 | 2 | 1 | 2 | 3 |
| 2 | 1 | 2 | 1 | 2 | 4 |
| 2 | 1 | 2 | 1 | 2 | 5 |
| 3 | 1 | 3 | 1 | 3 | 1 |
| 3 | 1 | 3 | 1 | 3 | 2 |
| 3 | 1 | 3 | 1 | 3 | 3 |
| 3 | 1 | 3 | 1 | 3 | 4 |
| 3 | 1 | 3 | 1 | 3 | 5 |
| 4 | 1 | 4 | 1 | 4 | 1 |
| 4 | 1 | 4 | 1 | 4 | 2 |
| 4 | 1 | 4 | 1 | 4 | 3 |
| 4 | 1 | 4 | 1 | 4 | 4 |
| 4 | 1 | 4 | 1 | 4 | 5 |
| 5 | 1 | 5 | 1 | 5 | 1 |
| 5 | 1 | 5 | 1 | 5 | 2 |
| 5 | 1 | 5 | 1 | 5 | 3 |
| 5 | 1 | 5 | 1 | 5 | 4 |
| 5 | 1 | 5 | 1 | 5 | 5 |
| 1 | 2 | 1 | 2 | 1 | 1 |
| 1 | 2 | 1 | 2 | 1 | 2 |
| 1 | 2 | 1 | 2 | 1 | 3 |
| 1 | 2 | 1 | 2 | 1 | 4 |
| 1 | 2 | 1 | 2 | 1 | 5 |
| 2 | 2 | 2 | 2 | 2 | 1 |
| 2 | 2 | 2 | 2 | 2 | 2 |
| 2 | 2 | 2 | 2 | 2 | 3 |
| 2 | 2 | 2 | 2 | 2 | 4 |
| 2 | 2 | 2 | 2 | 2 | 5 |
| 3 | 2 | 3 | 2 | 3 | 1 |
| 3 | 2 | 3 | 2 | 3 | 2 |
| 3 | 2 | 3 | 2 | 3 | 3 |
| 3 | 2 | 3 | 2 | 3 | 4 |
| 3 | 2 | 3 | 2 | 3 | 5 |
| 4 | 2 | 4 | 2 | 4 | 1 |
| 4 | 2 | 4 | 2 | 4 | 2 |
| 4 | 2 | 4 | 2 | 4 | 3 |
| 4 | 2 | 4 | 2 | 4 | 4 |
| 4 | 2 | 4 | 2 | 4 | 5 |
| 5 | 2 | 5 | 2 | 5 | 1 |
| 5 | 2 | 5 | 2 | 5 | 2 |
| 5 | 2 | 5 | 2 | 5 | 3 |
| 5 | 2 | 5 | 2 | 5 | 4 |
| 5 | 2 | 5 | 2 | 5 | 5 |

## Claims

1. A collection of barcoded nucleic acid molecules wherein each barcode comprises a sequence that is shared with the other barcodes in the collection and a sequence that is not shared with the other barcodes in the collection,
wherein the nucleotide residues that encode the non-shared sequence are interspersed with the nucleotide residues that encode the shared sequence such that the barcode comprises at least one uninterrupted sequence with the general formula I:
Xr-Ym-Xs
wherein
X and Y are nucleotide residues;
r, s and m are respectively the number of consecutive X and Y nucleotide residues;
m is 1, 2 or 3; and r and s are independently an integer of 1 or more; and
wherein the Xr and Xs nucleotide residues encode all or part of the shared sequence and the Yn nucleotide residues encode all or part of the non-shared sequence.

2. The collection of barcoded nucleic acid molecules of claim 1, wherein the barcode comprises two or more uninterrupted sequences with the general formula I.

3. The collection of barcoded nucleic acid molecules of claim 1 or claim 2, comprising at least one uninterrupted sequence with the general formula II:
Xr - Ym - Xs - (Z - Yn - Xt)p
wherein X, Y, r, s and m have the meaning as indicated in claim 1 and n is 1, 2 or 3; t is an integer of 1 or more, p is an integer of 1 or more, and Z is optional and when present is 1 or more non-barcode nucleotide residues.

4. The collection of barcoded nucleic acid molecules of claim 3, wherein p is 2, 3, 4 or 5.

5. The collection of barcoded nucleic acid molecules of any one of claims 1-4, wherein m=1 and the one or more of n, when present, are 1.

6. The collection of barcoded nucleic acid molecules of any one of claims 1-5, wherein r and s are independently an integer of 5 or more and the one or more of t, when present, are independently 5 or more.

7. The collection of barcoded nucleic acid molecules of any one of claims 1-6, further comprising a further barcode.

8. A collection of nucleic acid molecules comprising two or more collections of barcoded nucleic acid molecules of any one of claims 1-7.

9. The collection of barcoded nucleic acid molecules of any one of claims 1-8, wherein each of said molecules further comprises a different sequence of about 20-1000 nucleotides.

10. The collection of barcoded nucleic acid molecules of any one of claims 1-9, wherein the nucleic acid molecules of the collection are on average at least 25 nucleotides long.

11. The collection of barcoded nucleic acid molecules of any one of claims 1-10, wherein Y is an A or a T, preferably at least 2, 3, 4, 5 or 6 and preferably all instances of Y are an A or a T.

12. A collection of barcoded nucleic acid molecules of any one of claims 1-11, wherein the nucleic acid molecules are double stranded.

13. The collection of barcoded nucleic acid molecules of any one of claims 1-12, wherein the nucleic acid molecules are circular molecules.

14. A collection of at least two single or double stranded nucleic acid molecules that each comprise a head to tail repeat of a different member of the collection of barcoded nucleic acid molecules of any one of claims 1-13.

15. A method for sequencing a collection of nucleic acid molecules comprising providing a collection of nucleic acid molecules according to any one of claims 1-14 and sequencing said nucleic acid.

16. The method of sequencing according to claim 15, wherein said sequencing comprises long read sequencing.

17. The method of claim 15 or claim 16, further comprising determining the sequence of the collection of nucleic acid molecules.

18. The method of any one of claims 15-17, comprising aligning sequence reads on the basis of the sequence of the barcode in reads and determining a consensus sequence based on the alignment.

19. The method of any one of claims 15-18, wherein the sequencing comprises nanopore sequencing.
